# EUROPEAN PATENT SPECIFICATION

(11) **EP 1 616 494 B1**
(45) Date of publication and mention of the grant of the patent: **11.04.2007**
(21) Application number: 05015081.2
(22) Date of filing: 12.07.2005
(51) Int. Cl.: A43B 7/00, A61N 2/12

(54) **Hygienical shoes with mobile magnetic piece**
Hygienischer Schuh mit mobilen magnetischen Teilen
Chaussure hygiénique munie de pièces magnétiques mobiles

(30) Priority: 12.07.2004 CN 200420029313 U
(43) Date of publication of application: 18.01.2006
(73) Proprietor: Wang, Jian, Nankai District Tianjin 300384 (CN); Gong, Huimin, Nankai District Tianjin 300384 (CN)
(72) Inventor: Wang, Jian, Nankai District Tianjin 300384 (CN); Gong, Huimin, Nankai District Tianjin 300384 (CN)
(74) Representative: Beetz & Partner

(56) References cited:
- CN-A- 1 067 799
- CN-A- 1 113 817
- CN-A- 1 142 340
- US-A- 4 033 054
- PATENT ABSTRACTS OF JAPAN vol. 2002, no. 10, 10 October 2002 (2002-10-10) & JP 2002 177004 A (OHAMA HARUO; KOBAYASHI TAIJI; TAKAISHI GIICHI), 25 June 2002 (2002-06-25)

## Description

### Field of the Technology

This invention relates to a kind of hygienical shoes, more particularly to hygienical shoes with mobile magnetic piece.

### Background of the Invention

According to the meridian doctrine of the traditional Chinese medicine, there are some important meridians, such as the spleen channel, the urinary bladder channel, the stomach channel, the kidney channel, the liver channel and the gallbladder meridian, etc, and there are constituted a lot of important acupuncture points in the sole of foot. From the long-term practice of the traditional Chinese medicine, it has been proved that the massaging and stimulating for the meridians and acupuncture points in the sole of foot may dredge human body's meridians and activate human body's potential to attain the effect of dispelling illness, activating the channels and warming the internal organs. Therefore, "the reflect zone massotherapy of human body's thenar" is widely spread, and has become a health protection method and an auxiliary therapy for illness.

Recently, with the development of science and technology, a variety of hygienical shoes are provided to achieve the goal of massaging and stimulating the meridians and acupuncture points in the sole of foot. For example, a magnetic piece is provided onto the insole so that a magnetic field can stimulate a certain acupuncture point in the sole of foot. Though this kind of hygienical shoes can achieve the goal of magnetotherapy for the acupuncture point, the fixed magnetic piece set onto the insole generates only a static magnetic field for a certain acupuncture point in the sole of foot of the human body, and not a dynamic magnetic field. This restricts the effect of magnetotherapy. Thereby, this kind of hygienical shoes has some limitations.

In addition, in order to improve the magnetotherapy's effect in the sole of foot and to generate a dynamic magnetic field in the sole of foot of the human body, some shoes are provided with coils at their soles, thus, when the alternating current flows through the coils, a pulsing magnetic field for performing the magnetotherapy of dynamic magnetic field in the sole of foot of the human body is generated in the sole of foot of the human body. This kind of magnetotherapy shoes improve the effect of magnetotherapy, but the alternating current flowing through the sole's coil is necessary for the generation of a dynamic magnetic field, so that this kind of shoes can not be used for walking, but only for carrying out a magnetotherapy with a dynamic magnetic field in a fixed position only after settling in a certain place, pulling out the wire with a plug and inserting the plug into the power outlet.

All of the above-mentioned hygienical shoes can not carry out the magnetotherapy for the sole of foot with the static and dynamic magnetic fields generated respectively at the sole due to the rest and movement of foot and shoes when the human body stops and walks.

Document CN-A-1 067 799, shows an insole and lower sole. A magnetic part is provided for stimulating the sole of the foot to carry out magnetotherapy.

### Content of the Invention

The present invention is directed to a kind of hygienical shoes with at least one mobile magnetic piece which can carry out the magnetotherapy for the sole of foot with the static and dynamic magnetic field generated respectively at the sole due to the rest and movement of foot and shoes when the human body stops and walks.

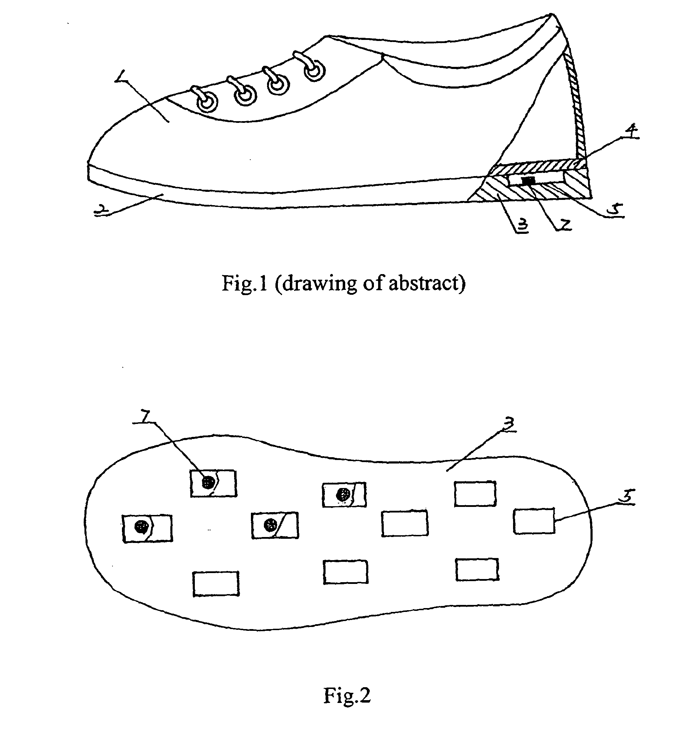

A shoe according to the present invention comprises a vamp and a sole, as shown in Fig. 1. The sole comprises a lower sole and an insole covering the upper surface of the lower sole. The upper surface of the lower sole is provided with grooves, and the mobile magnetic pieces are placed on the bottom of grooves, as shown in Fig. 2.

According to the present invention, there may be several grooves distributed all over the upper surface of the lower sole or allocated only in predetermined positions.

The bottom of the grooves according to the present inventions may be a horizontal plane or an inclined plane.

According to the present invention, the inside or bottom of the grooves may be provided with lining pieces which are made of a smooth-faced hard elastic sheet material; and staggered slits can be set on the longitudinal walls of the lining pieces.

The magnetic pieces of the present invention may be bead-like, columnar, or round-sheet shaped.

In walking with the shoes according to the present invention, when the foot lifts up to move forward, as the front of the shoes move upwards and onwards, the magnetic piece on the bottom of the groove will slide backwards to the back of the groove along the surface of the groove's bottom; and when the foot lifts up again after coming down to ground, the heel lifts up and, at the same time, the magnetic piece in the groove will slide to the front portion of the groove along the bottom surface of the groove. As such, the foot lifts up and comes down repeatedly and moves forward, the front and rear portions of the grooves change their relative positions continuously, and the magnetic pieces in the groove will slide or tumble depending on the obliquity of the bottom of the groove under the effect of gravity. The back-and-forth movement of the magnetic piece causes the movement of the magnetic field which, in turn, forms the dynamic magnetic field. When the dynamic magnetic field acts on the sole of foot of the human body, the massage of dynamic magnetic field enhances the effect of magnetotherapy for the sole of foot.

When resting, the static magnetic field of the magnetic piece may carry out the magnetotherapy with a static magnetic field for the sole of foot of the human body.

In a word, the present invention is a kind of hygienical shoes with mobile magnetic pieces, which can carry out the magnetotherapy for the sole of foot of the human body with the static and dynamic magnetic fields generated respectively at the sole due to the rest and movement of the foot and the shoe when the human body stops and walks.

### Brief Description of the Drawings

Fig. 1 is a schematic drawing of a hygienical shoe according to one embodiment of the present invention.
Fig. 2 is a top view of the lower sole of the shoe shown in Fig. 1.
Fig. 3 is a schematic drawing of the lower sole of an embodiment of the present invention.
Fig. 4 is a schematic drawing of the groove in the lower sole, the lining piece, the magnetic piece and the staggered slit according to an embodiment of the present invention.

In the figures: 1-vamp, 2-sole, 3-lower sole, 4-insole, 5-groove, 6-lining piece, 7-magnetic piece, 8-staggered slit.

### Detailed Description of the Invention

### Example 1:

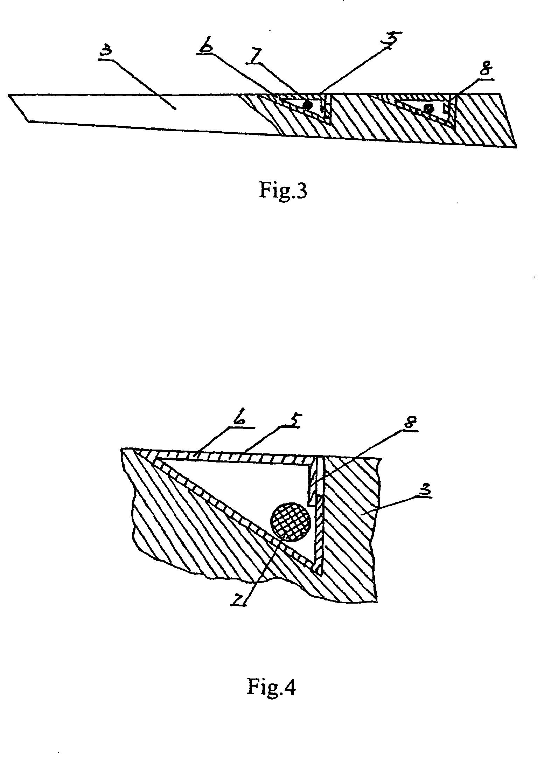

A travel shoe adopting the present invention comprises a vamp 1 made from sheepskin, and a sole 2 formed by a combination of an insole 4 and a lower sole, wherein the insole 4 is made from cowskin and the lower sole is made from polyurethane; 3x2 (6 in all) grooves 5 are provided on the upper surface of the lower sole 3; the bottoms of the grooves 5 are inclined planes with higher front portion and lower rear portion, respectively; the lining pieces 6 are set in the groove 5 along the upper, lower and back inside of the grooves, the longitudinal staggered slit 8 is set on the lining piece 6 of the back inside of the groove; and the mobile conglobate magnetic piece 7 is placed on the inner surface of the inside lining piece 6 of the groove's bottom, as shown in Fig. 3 and Fig. 4.

When the shoes according to the present invention are worn on the feet, in resting, the conglobate magnetic piece 7 will slide to the lower position of the inclined bottom of groove 5, i.e. the rear portion of the groove's bottom, along the inner surface of the lining piece under gravity; and the static magnetic field of the conglobate magnetic piece can stimulate directly the sole of foot of the human body.

While in walking, the heel lifts up after the foot comes down to the ground, and at this time, when the sole 2 is in the state of lower front and higher back, the conglobate magnetic piece 7 resting on the lining piece 6 of the back portion of the groove bottom will tumble to the front portion of the groove bottom along the inner surface of the lining piece 6 of the groove bottom under gravity; and when moving forwards, the groove 5 produces a forward acceleration along with the foot's movement, and due to the very small friction coefficient between the lining piece 6 and the conglobate magnetic piece 7, the conglobate magnetic piece 7 placed on the lining piece 6 of the front portion of the groove bottom will slide to the lower portion of the groove bottom 8 of the groove 5 under the backward counterforce and gravity.

Whenever the foot comes down to the ground, the lower sole 3 made from polyurethane will be subject to a compression deformation under the vertical downward force. At the same time, the longitudinal staggered slit 8 located on the lininig piece 6 will become shorter by interleaving along with the deformation of the lower sole 3, thereby the magnetic piece 7 can not press against the foot.

Again and again, whenever the foot moves forward a step, the conglobate magnetic piece 7 in each groove 5 of the lower sole 3 will move back and forth one time, and at the same time, bring the movement of magnetic field one time. The dynamic magnetic field moving to and fro can massage and stimulate the sole of foot of the human body directly.

According to the present invention, the static and dynamic magnetic field contacts the sole of foot of the human body directly and carries out the magnetotherapy for the sole of foot of the human body with two forms of magnetic field, what improves the magnetotherapy's effect of the sole of foot. In particular, the dynamic magnetic field, which is generated by the shoes themselves along with the motion of the foot and shoes when the human body is walking, can be used to carry out the magnetotherapy for the sole of foot of the human body.

## Claims

1. A hygienical shoe with at least one mobile magnetic piece (7), comprising a vamp (1) and a sole (2), wherein the sole (2) includes a lower sole (3) and an insole (4) covering the upper surface of the lower sole (3); at least one groove (5) is set on the upper surface of the lower sole (3), and the at least one mobile magnetic piece (7) is placed on the bottom of said at least one groove (7).

2. The hygienical shoe with at least one mobile magnetic piece (7) as set forth in claim 1, wherein several grooves (5) are provided.

3. The hygienical shoe with at least one mobile magnetic piece (7) as set forth in claim 1 or 2, wherein the inside or bottom of the at least one groove (5) is provided with lining pieces (6) which are made of a smooth-faced hard elastic sheet material.

4. The hygienical shoe with at least one mobile magnetic piece (7) as set forth in claim 3, wherein a staggered slit (8) is set on the longitudinal wall of the lining piece (6).

5. The hygienical shoe with at least one mobile magnetic piece (7) as set forth in one of the claims 1 to 4, wherein the groove's bottom is horizontal.

6. The hygienical shoe with at least one mobile magnetic piece (7) as set forth in claim 1, wherein the groove's bottom is inclined.

7. The hygienical shoe with at least one mobile magnetic piece (7) as set forth in claim 1, wherein the magnetic piece (7) has a bead-like shape.

8. The hygienical shoe with at least one mobile magnetic piece (7) as set forth in claim 1, wherein the magnetic piece (7) has a columnar shape.

9. The hygienical shoe with at least one mobile magnetic piece (7) as set forth in claim 1, wherein the magnetic piece (7) has a round-sheet shape.

## Patentansprüche

1. Hygienischer Schuh mit mindestens einem mobilen magnetischen Teil (7), mit einem Blatt (1) und einer Sohle (2), wobei die Sohle (2) eine untere Sohle (3) und eine Innensohle (4) aufweist, die die obere Oberfläche der unteren Sohle (3) bedeckt, mit mindestens einer Auskehlung (5) auf der oberen Oberfläche der unteren Sohle (3) und mindestens einem mobilen magnetischen Teil (7), welches auf dem Boden der mindestens einen Auskehlung (5) angeordnet ist.

2. Hygienischer Schuh mit mindestens einem mobilen magnetischen Teil (7) nach Anspruch 1, wobei mehrere Auskehlungen (5) vorgesehen sind.

3. Hygienischer Schuh mit mindestens einem mobilen magnetischen Teil (7) nach einem der Ansprüche 1 oder 2, wobei die Innenseite oder der Boden mindestens einer Auskehlung (5) mit Auskleidungsteilen (6) versehen ist, welche aus hartelastischem Folienmaterial mit glatter Oberfläche gefertigt sind.

4. Hygienischer Schuh mit mindestens einem mobilen magnetischen Teil (7) nach Anspruch 3, wobei ein abgestufter Schlitz auf der Längsseite des Auskleidungsteils (6) vorgesehen ist.

5. Hygienischer Schuh mit mindestens einem mobilen magnetischen Teil (7) nach einem der Ansprüche 1 bis 4, wobei der Boden der Auskleidung horizontal verläuft.

6. Hygienischer Schuh mit mindestens einem mobilen magnetischen Teil (7) nach Anspruch 1, wobei der Boden der Auskehlung geneigt ist.

7. Hygienischer Schuh mit mindestens einem mobilen magnetischen Teil (7) nach Anspruch 1, wobei das magnetische Teil (7) kugelförmig ist.

8. Hygienischer Schuh mit mindestens einem mobilen magnetischen Teil (7) nach Anspruch 1, wobei das magnetische Teil (7) zylinderförmig ist.

9. Hygienischer Schuh mit mindestens einem mobilen magnetischen Teil (7) nach Anspruch 1, wobei das magnetische Teil (7) eine flache runde Form aufweist.

## Revendications

1. Chaussure hygiénique avec au moins une pièce magnétique mobile (7), comprenant une empeigne (1) et une semelle (2), où la semelle (2) comprend une semelle inférieure (3) et une semelle intérieure (4) couvrant la surface supérieure de la semelle inférieure (3) ; au moins une rainure (5) est placée sur la surface supérieure de la semelle inférieure (3) et la au moins une pièce magnétique mobile (7) est placée sur le fond de ladite au moins une rainure (7).

2. Chaussure hygiénique avec au moins une pièce magnétique mobile (7), selon la revendication 1, dans laquelle plusieurs rainures (5) sont prévues.

3. Chaussure hygiénique avec au moins une pièce magnétique mobile (7), selon la revendication 1 ou 2, dans laquelle l'intérieur ou le fond de la au moins une rainure (5) est muni de pièces d'habillage (6) qui sont fabriquées en un matériau de feuille élastique dur à face lisse.

4. Chaussure hygiénique avec au moins une pièce magnétique mobile (7), selon la revendication 3, dans laquelle une fente décalée (8) est placée sur la paroi longitudinale de la pièce d'habillage (6).

5. Chaussure hygiénique avec au moins une pièce magnétique mobile (7), selon l'une des revendications 1 à 4, dans laquelle le fond de la rainure est horizontal.

6. Chaussure hygiénique avec au moins une pièce magnétique mobile (7), selon la revendication 1, dans laquelle le fond de la rainure est incliné.

7. Chaussure hygiénique avec au moins une pièce magnétique mobile (7), selon la revendication 1, dans laquelle la pièce magnétique (7) se présente sous la forme d'une bille.

8. Chaussure hygiénique avec au moins une pièce magnétique mobile (7), selon la revendication 1, dans laquelle la pièce magnétique (7) se présente sous la forme d'une colonne.

9. Chaussure hygiénique avec au moins une pièce magnétique mobile (7), selon la revendication 1, dans laquelle la pièce magnétique (7) se présente sous la forme d'une feuille circulaire.
